# EUROPEAN PATENT SPECIFICATION

(11) **EP 0 542 398 B1**
(45) Date of publication and mention of the grant of the patent: **21.11.2001**
(21) Application number: 92304303.8
(22) Date of filing: 13.05.1992
(51) Int. Cl.: A61K 35/78, A61K 7/48, A23L 3/3436, A61P 43/00

(54) **Rice extract useful as active oxygen scavenger**
Reisextrakt als Sauerstoffaufnehmer
Extrait de riz comme éliminateur d'oxygène actif

(30) Priority: 13.11.1991 JP 32402991
(43) Date of publication of application: 19.05.1993
(73) Proprietor: SOKEN Co., Ltd., Ayauta Gun, Kagawa Ken (JP)
(72) Inventor: Tokuyama, Yoshie, Kagawa-Ken (JP); Suzuki, Horoshi, Kagawa-ken (JP); Matsuo, Yoshihisa, Ayauta Gun, Kagawa Ken (JP); Takeuchi, Eiko, Ayauta Gun, Kagawa Ken (JP)
(74) Representative: Dixon, Donald Cossar

(56) References cited:
- EP-A- 0 027 514
- DE-A- 3 439 914
- GB-A- 2 195 889
- PATENT ABSTRACTS OF JAPAN vol. 13, no. 63 (C-568)1988
- AGRICULTURAL AND BIOLOGICAL CHEMISTRY vol. 49, no. 10, 1985, TOKYO JP pages 3085 - 3087 TOSHIHIKO OSAWA ET AL. 'ANTIOXYDATIVE DEFENSE SYSTEMS IN RICE HULL AGAINST DAMAGE CAUSED BY OXYGEN RADICALS'

## Description

The present invention relates to a method of preparing a novel active oxygen scavenger which is safe and can be used in many fields such as for medicaments, foods or cosmetics.

When a human being keeps his body healthy, active oxygen in the living body is always balanced with super oxygen dismutase (SOD), which is an active oxygen scavenging enzyme, and the concentration of active oxygen is maintained at an almost constant value. However, at present, due to an unbalanced diet, excessive stress, ageing or other factors, the formation of SOD is reduced and, on the other hand, due to smoking and air pollution, the content of active oxygen is increased.

As a result thereof, there can occur an excess of active oxygen in the body, which can cause various tissue lesions. In particular, in the case of an aged person, the reduction of the SOD activity and the increases of the concentration of active oxygen cause lesions such as articular rheumatism and the Behcet syndrome. Also, lipid peroxide formed by active oxygen becomes the main cause of modern diseases such as myocardial infarction, cerebral apoplexy, cataract, moth patches, freckles, wrinkles, diabetes mellitus, arteriosclerosis, stiff neck or feeling of cold.

Furthermore, even in those who are not aged, since active oxygen is particularly liable to form at the surfaces such as the skin, which are directly affected by environmental influences such as ultraviolet rays, lesions or traumas such as the formation of melanine dyes, moth patches or wrinkles are liable to occur.

Thus, the provision of SOD, for scavenging the excess active oxygen which causes the foregoing various lesions, has become the subject of research and it has been attempted to utilize SOD as medicament for the prophylaxis and treatment of these lesions or as an additive for cosmetics or foods. However, since SOD is unstable to heat and is inactivated by oral administration thereof as well as being very expensive, the attempt of scavenging active oxygen with SOD has not succeeded.

Therefore, active oxygen scavengers (including antioxidants having the same function as the SOD enzyme) have been investigated and active oxygen scavengers with a crude drug extract have been developed. However, such materials must be produced from specific raw materials and hence they are not only expensive but also cannot be stably supplied at present.

Thus, various investigations for the scavenging of active oxygen in the human body have been made since it was recognized that the aforesaid lesions are caused by active oxygen. Also, elderly people wish to live healthily in old age; and active oxygen scavengers also have been considered of use in beauty treatment.

Accordingly, it has been desired to develop an active oxygen scavenger which can safely be used in the human body, is inexpensive, excellent in its effect of scavenging active oxygen which causes various lesions as described above, can be easily produced, and also can be stably supplied.

We have investigated various vegetable components with rice as their main subject. During the investigations, it has been discovered that rice contains components showing useful functions. Thus, the active oxygen scavenging effect with the water extract or the organic solvent extract of rice was measured, and as the result thereof, it has been discovered that the extract has a very remarkable active oxygen scavenging effect and based on the discovery, the present invention has been accomplished.

According to one aspect of the present invention there is provided a method of preparing a scavenger of active oxygen, which comprises the steps of grinding polished rice, adding warm water and liquefying amylase to the ground polished rice, stirring the mixture well, gradually raising the temperature of the mixture to boiling, extracting under boiling, cooling the extract, pressing the mixture in a pressing apparatus, adding a cultured yeast solution to the pressed out extract, keeping the mixture at a constant temperature to carry out alcoholic fermentation, and filtering the fermented product.

According to another aspect of the present invention there is provided a medicament, cosmetic or food containing a scavenger of active oxygen prepared according to the above method.

Since rice has been used from old as a staple food especially in Japan, and it has thus been established that the safety of rice is of the highest, the active oxygen scavenger which is safe for the human body can be provided easily and at a low cost.

In the present invention, in the case of extracting rice with water or an organic solvent, when rice is ground or powdered, the surface area of the rice is increased, whereby the extraction efficiency is greatly improved. This may be practiced by any usual method using a grinder or a rice-cleaning machine. In this invention, the rice need not be ground, but in such a case, it takes a long time to decompose the rice tissue and extract it.

To perform extraction with water, water is added to rice as it is, or preferably to ground or powdered rice. As to the amount of added water, an amount of from 2 to 5 times the amount of the rice is enough. After adding the water which can be hot water, to rice, the mixture is heated and when the mixture boils, the extraction is finished.

After finishing the extraction, by compressing and filtering the extract, a clear extract is obtained.

The effective components extracted from rice have not yet been clarified, but since it has been confirmed that the unknown effective components are stable to heat, a high temperature is efficiently employed as the extraction temperature at the water extraction. However, when the temperature is low, the extraction of rice can be sufficiently practiced by carrying out the extraction for a long time. When the extraction temperature is 40°C or lower, it is necessary to carry out the extraction in an acidic state or an alkaline state, or by adding an antiseptic to the extraction system so that the rice is not rotted. The extraction time may be a few minutes in the case of the boiling extraction but when the extracting temperature is an intermediate (i.e., from the boiling point to about 40°C), an extraction time of from a few hours to a whole day and night is required. In the case of a lower temperature, an extraction time of from a few days to one month is required, although it depends upon whether the rice is ground or powdered. However, in the latter case, it is more effective to heat the system finally.

In the case of water extraction, the most severe problem is the gelatinisation phenomenon of the rice. If rice is gelatinized, not only is the extraction efficiency reduced but also the practical operation becomes very difficult. For preventing the occurrence of this trouble, it is advantageous to decompose the starch by adding amylase thereto to cause reaction with the starch or acidifying the starch by addition of hydrochloric acid. By employing such method, the foregoing problem can be adequately solved and there is no problem in practical operation.

Also, since it has been confirmed that the effective component extracted from rice is stable to acid and alkali, it is also possible to carry out an extraction of the rice by use of an acid or alkali. Furthermore, in the case of the extraction with water, the rice may be pre-treated with an acid or an alkali or with an enzyme (e.g., amylase) which acts on the tissue of the rice; such pre-treatment is considered to be effective since the effective components of the rice become liable to be extracted by the application of the pre-treatment.

Moreover, we have found that by an organic solvent extraction of rice, the extract of the present invention can also be obtained. This is very effective for making clear the effective components of this invention and for extracting the effective components or rice at a concentrated state as well as for utilizing the effective components by compounding with other component(s) insoluble in water. If an organic solvent is used, it is preferred to use a solvent which is safe when it is administered to a human being, such as ethanol.

The rice extract obtained may be subjected to a fermentation treatment such as alcoholic fermentation or lactic acid fermentation.

Rice is used as a staple food; it has never been considered to use rice as an active oxygen scavenger. Also, rice has been used as the raw material for rice wine (sake), rice spirit (shochu) and vinegar, but it has never been considered or practiced to make a rice extract. This is considered to be because on attempting to make such extract by heating, the rice is gelatinized and hence the making of an extract of rice by heating has been very difficult by conventional methods.

However, in the present invention, we can use an organic solvent extraction, an acid or alkali extraction, or in the case of extraction with water, an enzyme pretreatment to facilitate the extraction.

By carrying out an appropriate extraction as described above, the effective components of rice as a very excellent active oxygen scavenger can first be extracted.

In the accompanying drawings:
Fig. 1 is a graph showing the results of applying a single coating test using a moisture meter to measure the moisturizing effect of the scavenger of the present invention;
Fig. 2 is a graph showing the results of applying a water sorption-absorption test before using the scavenger of the present invention and after using the scavenger for two weeks;
Fig. 3 is a graph showing the effect on the amount of skin lipids of coating the scavenger of this invention after washing a face;
Fig. 4 is a graph showing measurement of the friction coefficient of untreated skin;
Fig. 5 is a graph as in Fig. 4 after coating the scavenger of the present invention for one week;
Fig. 6 is a graph of the ultraviolet absorption spectrum of the scavenger of the present invention;
Fig. 7 is a magnified view showing the states of the furrows and raised areas of the skin of a woman subject 21 years old;
Fig. 8 is a view as in Fig. 7 of a woman subject 71 years old;
Fig. 9 is a view as in Fig. 8 wherein the skin was coated with the scavenger of the present invention for one week;
Fig. 10 is a view as in Fig. 7 of skin of a 40 year old woman;
Fig. 11 is a view as in Fig. 10 wherein the skin was coated with the scavenger of the present invention for one week;
Fig. 12 is a graph showing the result of a detergent foaming power test; and
Fig. 13 is a graph showing the result of determining the storage effect by the scavenger of the present invention on a drink made from fermented rice (amazake).

The scavenging effect of the active oxygen scavenger of the present invention (hereinafter referred to as the scavenger of the present invention) is described below.

First, the effect of the scavenger of the present invention as a super oxide scavenger was determined by an enzyme method.
The following were prepared:-

### Preparation of Reagent

(1) 0.05 M Na₂CO₃ Buffer Solution (pH 10.2)
(2) In the buffer solution (1) was dissolved 45.6 mg of xanthin to provide 100 ml of a 3 mM xanthin solution.
(3) In distilled water was dissolved 111.7 mg of ethylene diamine tetraacetic acid.2Na to provide 100 ml of a 3 mM EDTA solution.
(4) In distilled water was dissolved 15 mg of bovine serum albumin (made by Sigma Co, Japan) to provide 10 ml of a BSA solution.
(5) In distilled water was dissolved 61.32 mg of nitro blue tetrazolium (NBT) to provide 100 ml of a 0.75 mM NBT solution.
(6) Xanthin oxidase was dissolved in distilled water to a concentration such that the absorbance in the air test of the operation method (analysis method) described below was in the range of from 0.2 to 0.23.
(7) In distilled water was dissolved 102.26 mg of CuCl₂.2H₂O to provide 100 ml of a CuCl₂ 6 mM solution.

### Operation Method

(1) In a test tube was placed 2.4 ml of the Na₂CO₃ buffer solution, and then 0.1 ml of the xanthin solution, 0.1 ml of the EDTA solution, 0.1 ml of the BSA solution and 0.1 ml of the NBT solution described above were added to the buffer solution.
(2) Then, 0.1 ml of a solution of a sample was added to the mixture, and after allowing the resultant mixture to stand for 10 minutes at 25°C, 0.1 ml of the xanthin oxidase solution described above was added thereto followed by stirring quickly and the mixture was incubated.
(3) After 20 minutes since the incubation, 0.1 ml of the CuCl₂ solution described above was added thereto to stop the reaction and the absorbance was measured at 560 nm.
(4) For comparison, the same test was carried out on 0.1 ml of an aqueous solution of super oxide dismutase (Cu,Zn-type SOD, activity 3000 to 4000 units/mg, made by Wako Junyaku K.K.) in place of the sample and the value obtained was defined as a super oxide scavenging ratio of 100%.
(5) Also, the same test was carried out using distilled water in place of the sample to provide a blank.

The measured results are shown in Table 1.

**Table 1**

| | Water Extract | Organic Solvent Extract | Alcoholic Fermentation After Water Extract | SOD |
|---|---|---|---|---|
| Super Oxide Scavenging Ratio (%) | 53 | 56 | 57 | 100 |
| Notes: The water extract was as obtained in Comparative Example 1. The organic solvent extract was as obtained in Comparative Example 3. The alcohol fermentation product after water extraction was as obtained in Example 1. | | | | |

As is clear from the above results, it can be seen that the water extract and the organic solvent extract have a super oxide scavenging effect.

Then, the heat stability of the scavenger of the present invention was determined. Each of the scavenger of the present invention obtained in Comparative Example 1 and SOD was heat-treated for 10 minutes at 70°C and the super oxide scavenging property of each was determined by the method described above.

The results obtained are shown in Table 2 below.

**Table 2**

| | Water Extract | Organic Solvent Extract | Alcoholic Fermentation After Water Extract | SOD |
|---|---|---|---|---|
| Super Oxide Scavenging Ration (%) | 56 | 51 | 59 | 0 |
| Note: Each extract was one obtained in each example as in Table 1. | | | | |

As is clear from the above results, it can be seen that SOD is unstable to heat, while the scavengers of the present invention are all excellent in heat stability. Thus, it can be said that the effective components of the present invention capable of scavenging active oxygen are excellent in stability to heat.

Furthermore, the water extract obtained in Comparative Example 1 was concentrated thrice in a rotary evaporation, and the super oxide scavenging property of the concentrate was determined. Measurement of the super oxide scavenging ratio was carried out by the foregoing method. The results are shown in Table 3.

**Table 3**

| | Concentrate of Water Extract | SOD |
|---|---|---|
| Super Oxide Scavenging Ratio (%) | 93 | 100 |

As is shown above, by concentrating the scavenger of the present invention, the concentrated scavenger has almost the same super oxide scavenging effect as that of SOD.

Since the scavenger of the present invention shows a very remarkable active oxygen scavenging effect and is safe from a health viewpoint, it can be utilized for medicaments, cosmetics or foods, and these uses are explained below.

As a medicament, the scavenger of this invention can be used as an anti-ulcer agent. The test procedure for determining the anti-ulcer activity of the scavenger of this invention and the result thereof are as follows.

First, the activity of the scavenger of this invention to an ulcer induced by a restraint-cold stress in oral administration was determined. The procedure was carried out according to the Watanabe et al method. That is, after fasting a ddy male mouse of 8 week age for 24 hours, 0.32 ml/mouse of the scavenger of this invention obtained in Comparative Example 1 shown below was orally administered, after 30 minutes, the mouse was placed in a stress gauge, and the mouse was immersed except for its head in water at a temperature of 15°C so as to apply to it a restrained-cold stress. After 5 hours, the cervical vertebra was dislocated to slaughter the mouse, and then, the stomach thereof was removed. Thereafter, 1.5 ml of an aqueous 1% formalin solution was injected into the stomach, the stomach was further immersed in the aqueous 1% formalin solution to lightly fix the stomach tissue, and the stomach was allowed to stand as it was for 24 hours. Thereafter, the stomach was incised along its greater curvature, the length (mm) of the injury caused at the gastric glands was measured and the sum total thereof per one mouse was shown as the ulcer coefficient. Also, as a control, a male mouse of the same age orally administered with an isotonic sodium chloride solution 30 minutes before placing the mouse in the stress gauge was used. In the test, a total of 15 mice were used in each case.

The results obtained are shown in Table 4 below.

**Table 4**

| | Administered Amount (ml/mouse) | Number of Test Mice | Mean Ulcer Coefficient |
|---|---|---|---|
| Isotonic Sodium Chloride Solution | 0.3 | 15 | 65.4 |
| Scavenger of the invention | 0.3 | 15 | 29.1 |

As shown in Table 4, it can be seen that while the mean ulcer coefficient of the mice administered with an isotonic sodium chloride solution as a control is 65.4, the mean ulcer coefficient of the mouse administered with the scavenger of the present invention is 29.1, which shows clearly that the oral administration of the scavenger of the present invention is effective as an anti-ulcer agent for ulcers induced by restraint-cold stress. It has thus been found that the scavenger of the present invention has an effective function as an anti-ulcer agent by directly acting on the mucous membranes of the stomach and intestines.

Then, the effect of injection under the skin of the scavenger of the present invention on ulcers induced by restraint-cold stress was determined. The method was carried out as in the case of the oral administration. That is, 15 mice each injected hypodermically with 0.3 ml/mouse of the scavenger of the present invention and 15 mice each injected with an isotonic sodium chloride solution were allowed to stand for 30 minutes; thereafter, they were placed in a stress gauge, and after applying thereto a restraint-cold stress, the effectiveness against ulcers induced by restraint-cold stress of the administration of the scavenger of the present invention was determined.

The results obtained are shown in Table 5 below.

**Table 5**

| | Administered Amount (ml/mouse) | Number of Test Mice | Mean Ulcer Coefficient |
|---|---|---|---|
| Isotonic Sodium Chloride Solution | 0.3 | 15 | 43.0 |
| Scavenger of the Invention | 0.3 | 15 | 22.7 |

As is clear from the results shown in Table 5, it can be seen that the mean ulcer coefficient of 15 mice each administered hypodermically with 0.3 ml/mouse of an isotonic sodium chloride solution is 43.0, while the mean ulcer coefficient of 15 mice each administered likewise hypodermically with the scavenger of the present invention is 22.7, which show the effectiveness of the scavenger of the present invention as an anti-ulcer agent by hypodermic administration.

From the results described above, it has been found that the scavenger of the present invention is effective on ulcers caused by stress in both oral and hypodermic administration.

Then, the effectiveness of the scavenger of the present invention on an ethanolic ulcer, which is an ulcer occuring by direct action on the mucous membrane of the stomach, as an anti-ulcer agent was determined in an oral administration. Since it has been confirmed as described above that the scavenger of the present invention is effective on stress ulcers in oral administration and in stantaneous administration, oral administration only was carried out in this test. The test on the ethanolic ulcers was carried out according to Robert et al's method. That is, after fasting a Wister/ST series male rat of eight weeks age for 24 hours and abstaining the rat from water for 16 hours, 1.0 ml/rat of a solution obtained by dissolving 5 g of the scavenger of the present invention obtained in Comparative Example 2 in 10 ml of an isotonic sodium chloride solution was orally administered to the rat, and after 30 minutes the cervical vertebra was dislocated to slaughter the rat, and then the stomach thereof was removed. Thereafter, 10 ml of an aqueous 1% formalin solution was injected into the stomach, the stomach was immersed in the aqueous 1% formalin solution to lightly fix the stomach tissue, and the sum total of ulcers generated at the gastric glands was measured as the ulcer coefficient, as in the case of mice. Also, in this case, rats orally administered with an isotonic sodium chloride solution were used as a control. In the test, 15 rats were used for each test.

The results obtained are shown in Table 6 below.

**Table 6**

| | Doses (ml/rat) | Number of Rats Tested | Mean Ulcer Coefficient |
|---|---|---|---|
| Isotonic Sodium Chloride Solution | 1.0 | 15 | 48.1 |
| Solution of Scavenger of the Invention | 1.0 | 15 | 19.1 |

As is clear from the results shown in Table 6, it can be seen that while the mean ulcer coefficient in the rats administered with an isotonic sodium chloride solution as a control is 48.1, the mean ulcer coefficient in the rats orally administered with 1.0 ml/rat of the scavenger of the present invention is 19.1, and hence the scavenger of the present invention is an effective anti-ulcer agent on an ethanolic ulcer caused by directly action on the mucosa membrane of a stomach.

Also, the scavenger of the present invention can be utilized as a therapeutic agent for the skin. The scavenger of the present invention was coated twice morning and evening to the affected parts of human subjects having various kinds of skin disorders, and after continuing the coating therapy daily for one month the affected parts were examined.

The results are shown in Table 7 below.

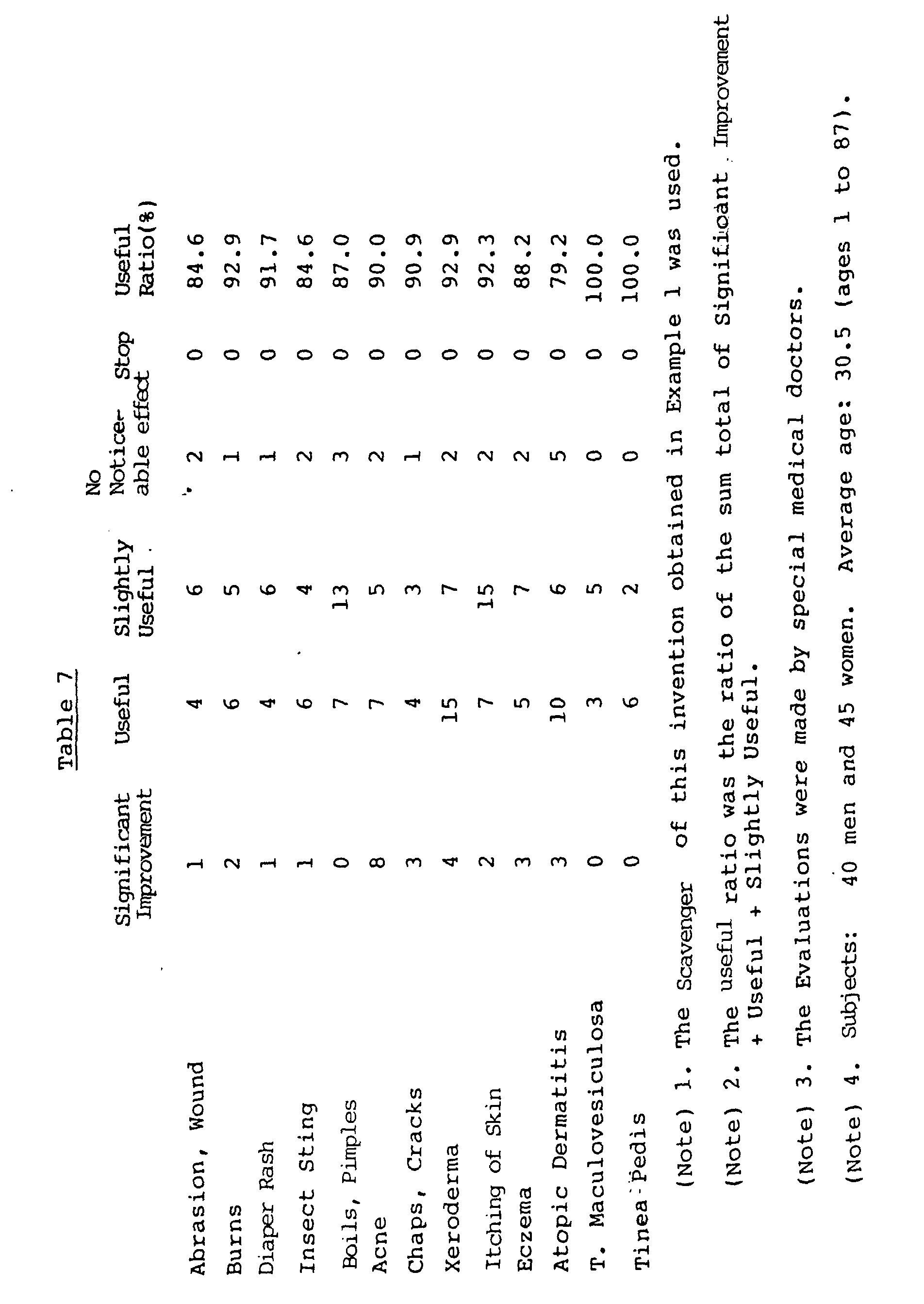

As shown in above Table 7, since the scavenger of the present invention has the effects of various skin therapeutic agents, it can be seen that the scavenger has a fibroblast-activating activity and further an antimicrobial activity.

Also, since the scavenger of the present invention is useful against xeroderma, acne and similar conditions it can be seen that the scavenger also has a moisturizing effect and an effect of restraining the increase of skin lipids. The test method of practically determining the moisturizing effect and the effect of restraining the increase of skin lipids and the results thereof are as follows.

First, for illustrating the power of the moisturizing effect of the scavenger of the present invention, a single coating test was carried out using a moisture meter (SKICON 200, trade name, made by Soken Co., Ltd.). As the measuring conditions, a room temperature of 20°C and a relative humidity of 65% were selected, and the subjects were rested under the foregoing conditions for about 10 minutes before the measurement. As the test areas, the flexural sides of forearms (both sides of the arm) having no efflorescence of the skin were selected.

The test was carried out on five subject suffering from a xeroderma.

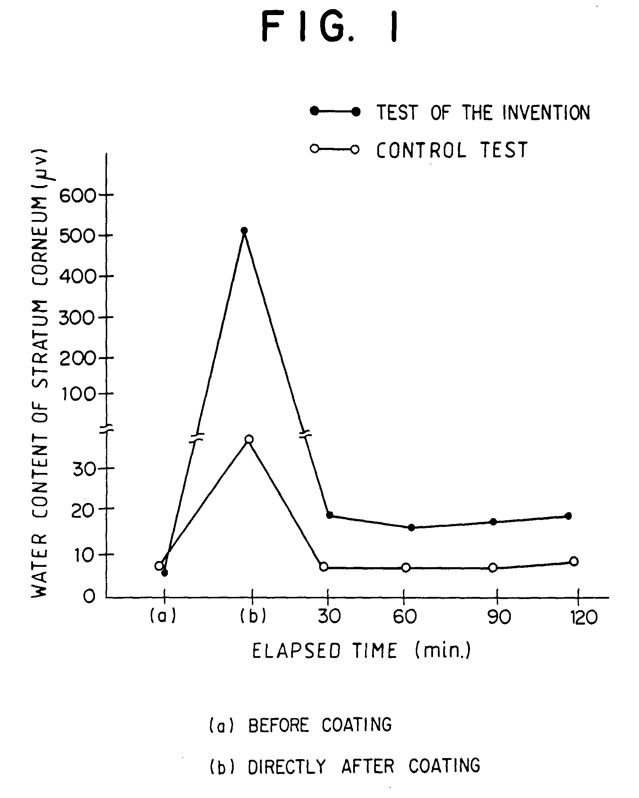

The mean values of the changes of the water content of the main test (using the scavenger of the present invention obtained in Comparative Example 1 and the control test (using water) read from the moisture meter are shown in Fig. 1 of the drawings.

The measurement method of the single coating test was as follows.
1) A test area and a control area each of size 5 x 5 cm² were selected on the bent forearm (antebrachial) side of each subject.
2) The water content of the stratum corneum of each area was measured.
3) The water content of the stratum corneum was measured directly after coating the sample or after 30 minutes, 60 minutes, 90 minutes or 120 minutes since coating the sample.

From the results shown in Fig. 1, in the case of coating the scavenger of the present invention, for the water content of the stratum corneum an increase of about 10 times that in the control was observed directly after coating. Also, it was seen that in the case of after 30 minutes to 120 minutes since coating, the area coated with the scavenger of the present invention kept its water in an amount of from 2 to 3 times that in the control case up to 120 minutes.

Then, for numerically assessing the medical treatment effect of a xeroderma by the scavenger of the present invention, an in vivo water sorption-desorption test before using the scavenger of Comparative Example 1 of this invention and after 2 weeks since the use of the scavenger was carried out using a moisture meter (SKICON 200). The subjects were the same five subjects used for obtaining the mean values shown in Fig. 1 and the measurement condition carried out in this case was the same as that in the foregoing single coating test.

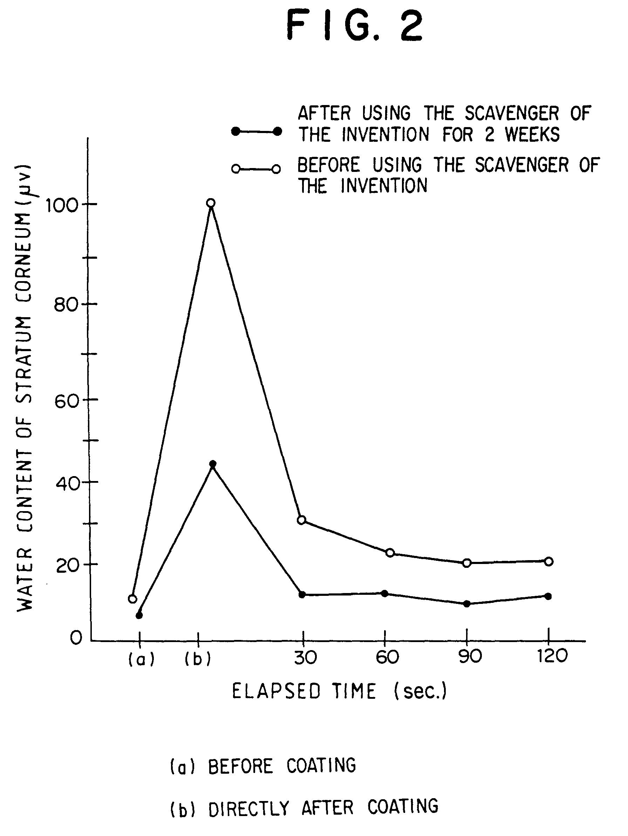

In addition, for the evaluation of the effect, a control test (on the skin area which was uncoated with the scavenger of this invention) was always carried out such that the evaluation of the main test was not influenced by the seasonal change of the water content of the stratum corneum of the body. The water content of the stratum corneum, as shown by the mean value of the five subjects, is shown in Fig. 2.

The measurement method in the in vivo water sorption-desorption test was as follows.
1) The water content of the stratum corneum at the test area was measured.
2) One drop of distilled water was placed on the test area and after 10 seconds the water drop was completely wiped away with a dry gauze.
3) The water content of the stratum corneum was measured directly after the wiping away and 30 seconds, 60 seconds, 90 seconds and 120 seconds after the wiping away.

As is shown in the graph of Fig. 2, it can be seen that by coating the scavenger of the present invention, both the water absorbing faculty of the skin (the difference in value of the water content of the stratum corneum at the time of the water loading and before water loading) and the water-keeping faculty (the curve showing the change of the water content of the stratum corneum up to 120 seconds since water loading) are simultaneously improved.

That is, in the skin before coating the scavenger of the present invention, the water content of the stratum corneum before water loading is very low (4.2 on an average) and the water absorbing faculty (40.8 on an average) is considerably lowered. Also, in regard to the water-keeping faculty, the stratum corneum of a normal man gradually releases absorbed water, while in the case of the test subjects, at 30 seconds after water loading the state of the stratum of the skin returned to the value before water loading. These results show that in the pathological stratum corneum, the water-absorbing faculty, the water-keeping faculty, and the barrier function are all lowered. On the other hand, it has been confirmed that in the skin after being coated with the scavenger of the present invention, the water content of the stratum corneum before water loading and its water-absorbing faculty increase from 2 to 3 times and also the water-keeping faculty is considerably improved to almost the same as that of a normal man.

From the above facts, it is seen that the scavenger of the present invention has an excellent effect in improving the water-containing state and the barrier function of the pathological stratum corneum. Also, by evaluating the scavenger of the present invention together with the moisturizing effect obtained by the single coating test, it can be seen that the scavenger of the present invention has the moisturizing effect of imparting to the stratum corneum the properties of increasing the water absorbing faculty and water-keeping faculty of the stratum corneum, absorbing a large amount of moisture from outside and retaining water once absorbed.

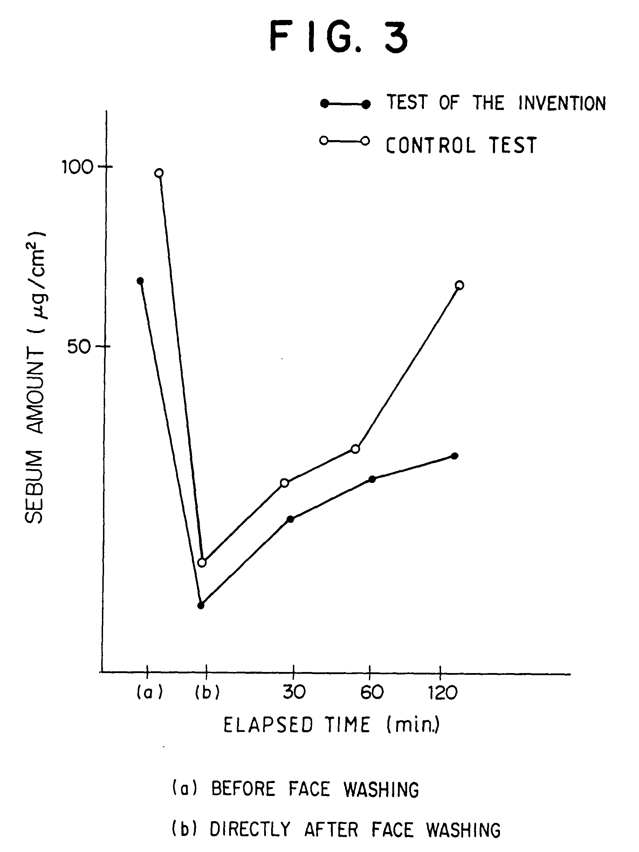

Furthermore, for experimentally illustrating the effect of the scavenger of Comparative Example 1 of the invention in inhibiting secretion of skin lipids, the change of the amount of skin lipids after face washing was measured. For this, five subjects randomly selected from the subjects used for the skin disorder results shown in Table 7 above were used, and the mean value of the change of the amount of skin lipids in the main test (coating the scavenger of this invention after face washing) and in a control test (face washing only) is shown in Fig. 3.

As shown in Fig. 3, it has been found that by coating the scavenger of the present invention, the increase of the amount of sebum is considerably restrained. Because of the effect of the scavenger of this invention in restraining secretion of sebum the scavenger is effective in the prophylaxis and treatment of acne.

Moreover, the following test shows that coating of the scavenger of this invention on skin as a cosmetic has the effect of smoothing and refining the skin. The scavenger of this invention was coated on a right arm area of each subject twice a day for one month and the area coated with the scavenger of this invention was measured by a kinematic friction meter. As a control, the same area of the left arm was used. The test was made on six subjects.

The measurement conditions were as follows.

| | |
|---|---|
| Temperature | 25°C |
| Humidity | 60% |
| Sensor | KES-SE Friction Tester SR-R Type (0.5 mm piano wire used) |
| | |
| Friction Static Load | 50 gf |
| Measuring Rate | 1 mm/sec. |
| Measuring Distance | 30 mm (integrated effective range along the abscissa between 2 and 0 cm: 20 mm) |

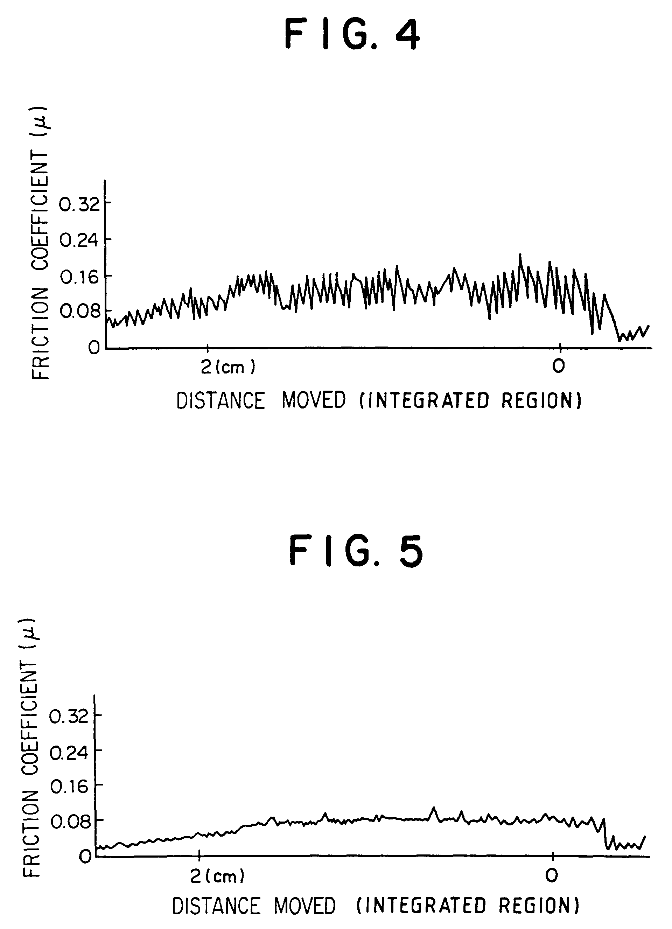

The results are shown in Figs. 4 and 5.

As shown in Fig. 4, in the area of the left arm uncoated with the scavenger of the present invention, the MMD (coefficient of variation) was 0.0172 but in the area of the right arm coated with the scavenger of the present invention for one month, the MMD was reduced to 0.0042 as shown in Fig. 5. The mean value of each of the six subjects was almost the same. This is considered to be because the variation in the unevenness of the surface is lowered, from which it has been found that the texture of the skin becomes fine.

In addition, when the MIU (friction coefficient) was also determined at the same time, MIU before coating the scavenger of the present invention was 0.123 but was lowered to 0.073 for skin after coating with the scavenger of the present invention for one month. Thus, it has been found that the scavenger of this invention also has an effect of the smoothing skin.

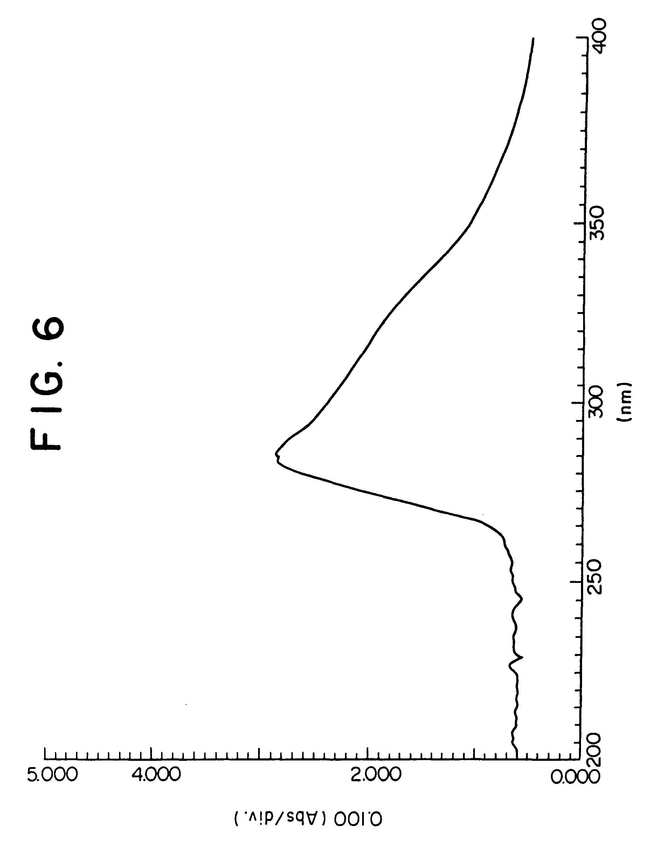

Furthermore, to graphically illustrate the ultraviolet absorption effect of the scavenger of the present invention, its absorption spectrum was measured; the result is shown in Fig. 6.

From the graphs shown in Fig. 6, it is seen that the scavenger of the present invention is particularly excellent in its absorption effect in the low wavelength region (about 320 to 340 nm), in the UVB region of from 280 to 320 nm and in the UVA region of from 320 to 380 nm.

Since the scavenger of the present invention does not have a sufficient absorption near the UVB region, it may be considered that the ultraviolet absorption effect of the scavenger is insufficient; but it is said that the shortest wavelength of ultraviolet rays reaching the surface of the earth is 295 nm and the longest wavelength thereof is from 320 nm to 330 nm, and hence that the ultraviolet absorption faculty in practice is sufficient. The ultraviolet absorption faculty of the scavenger of the present invention is about thrice that of a currently used crude drug extract and crude drug components (oil-soluble liquorice extract P-U (Spanish juice), licochal cone A, baicalein, γ-oryzanol) having a natural ultraviolet absorption property. Thus, the scavenger of the present invention has excellent ultraviolet absorption.

Furthermore, for illustrating the good whitening action of the scavenger of the present invention, a test of its tyrosinase activity hindering action was carried out.

The test procedure was as follows. That is, 1 ml of a substrate solution (aqueous 0.04% tyrosine solution) and 1 ml of a buffer solution (Macllvaine Buffer, pH 6.8) were correctly placed in an absorption cell and after adding thereto 1 ml of water or 1 ml of the scavenger of the present invention obtained in Comparative Example 1, the resultant mixture was mixed by stirring at 35°C. After 5 minutes, the absorbance scale was set to the wavelength 475 nm to carry out a zero correction. Then, 0.02 ml of a tyrosinase solution (obtained by dissolving 5.3 mg of tyrosinase in an aqueous 0.9% NaCl solution) was added and the mixture was immediately stirred and incubated.

The absorbance was measured every 3 minutes; the results obtained are shown in Table 8 below.

**Table 8**

| | Absorbance | |
|---|---|---|
| Minutes | Water | Scavenger of the the Invention |
| 0 | 0.007 | 0.016 |
| 3 | 0.063 | 0.057 |
| 6 | 0.152 | 0.081 |
| 9 | 0.243 | 0.097 |
| 12 | 0.329 | 0.112 |
| 15 | 0.419 | 0.127 |
| 18 | 0.497 | 0.141 |
| 21 | 0.560 | 0.152 |
| 24 | 0.617 | 0.163 |
| 27 | 0.637 | 0.175 |
| 30 | 0.646 | 0.184 |

From the measurement results in Table 8, it can be seen that the scavenger of the present invention has a tyrosinase activity hindering action. Thus, it can be said that the scavenger of the present invention has a beautiful whitening action.

Then, for determining the rejuvenating action of the skin by the scavenger of the present invention, the scavenger was coated on the back of the hand of each of six women subjects of 70 to 80 years old for one week, the coated portion was sampled by the SUMP (Suzuki's Universal Micro-Printing) method, a x 100 microphotograph of the image obtained by the SUMP method was taken, and the result was compared with that of the uncoated skin.

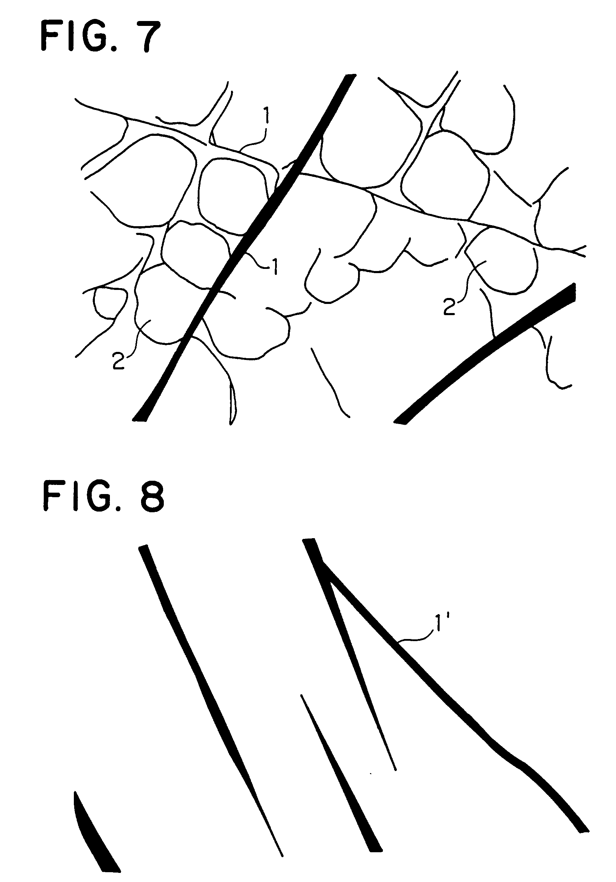

As shown in Fig. 7, in the skin of a woman subject of twenty one years old, furrows 1 are clearly observed running in many directions as well as raised areas 2. However, as shown in Fig. 8, in a seventy one year old woman, furrows 1' only are observed in one direction and no raised areas are observed. It is said that with age, the skin loses its resilience, the furrows of the skin become shallow by stretching of the skin, and the raised areas gradually disappear, and these changes can be confirmed by the test.

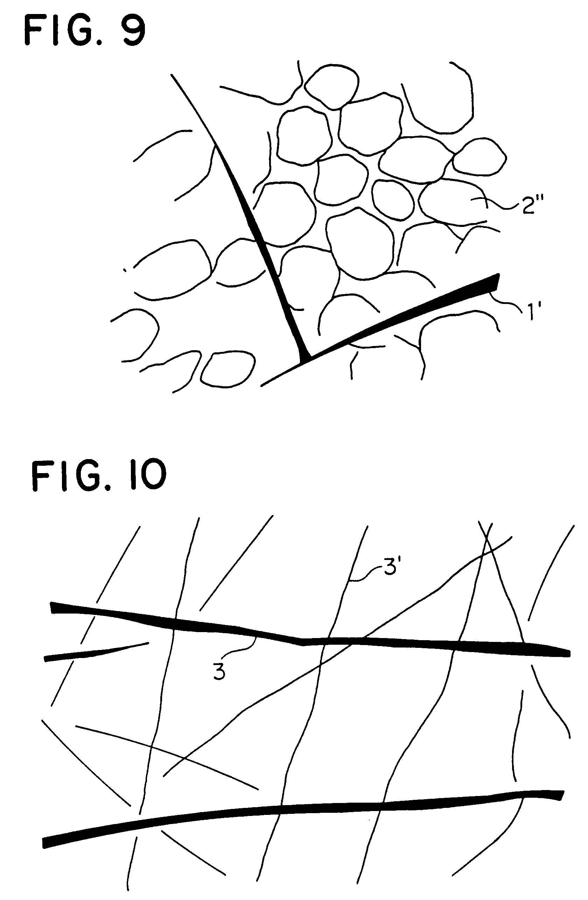

On the other hand, when the scavenger of the present invention was coated on the skin for one week, as shown in Fig. 9, the furrows which were observed in one direction only before coating the scavenger of this invention were clearly observed (1') in many directions. Moreover, the raised areas 2' which were not observed in the seventy one years woman were clearly observed and the her skin became almost the same as the skin of the twenty one years old woman; and as to sensation, the resilience was increased. This phenomenon was also observed similarly on the remaining subjects. Thus, it can be said that by using the scavenger of the present invention, the essential function of the skin is recovered and clear and well-proportioned micro fingers appears inside the raised areas. The scavenger of the present invention thus has a rejuvenating action on the skin.

Then, for illustrating the ageing-preventing action of the scavenger of the present invention, the scavenger of the Comparative Example 1 of the invention was continuously coated on the back of the hand of each of three woman subjects each forty years old for one week and the coated portion was microscopically observed by the SUMP method.

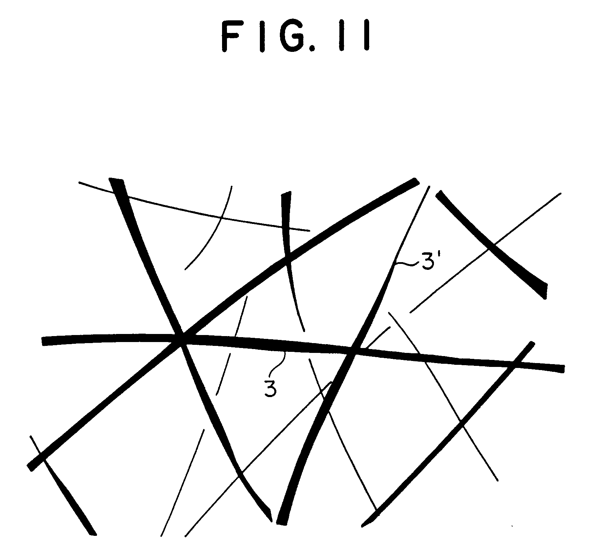

As shown in Fig. 10, in the skin uncoated with the scavenger of the present invention, the furrows 3 in one direction only were clearly observed and the furrows 3' in the direction crossing the direction of the furrow 3 were indistinct. On the other hand, in skin continuously coated with the scavenger of the present invention for one week, as shown in Fig. 11, the furrows 3' which were indistinct clearly appeared, and fine furrows were observed. This phenomenon similarly appeared in two other subjects. Thus, the scavenger of the present invention was an action for preventing ageing of the skin.

Moreover, as described above, the scavenger of the present invention has a moisturizing effect so that it is capable of being used in an medicament. Thus, the scavenger of this invention satisifies all the requirements of a cosmetics base and can be used in a variety of materials, such as creams, milky lotions, face lotions, cleansing creams, packs or soaps.

Also, by drinking the scavenger of this invention, the same effects as described above are obtained.

Furthermore, we show in the following test that by adding the scavenger of the present invention to kitchen detergents, washing detergents, etc., the washing power and the foaming power are increased.

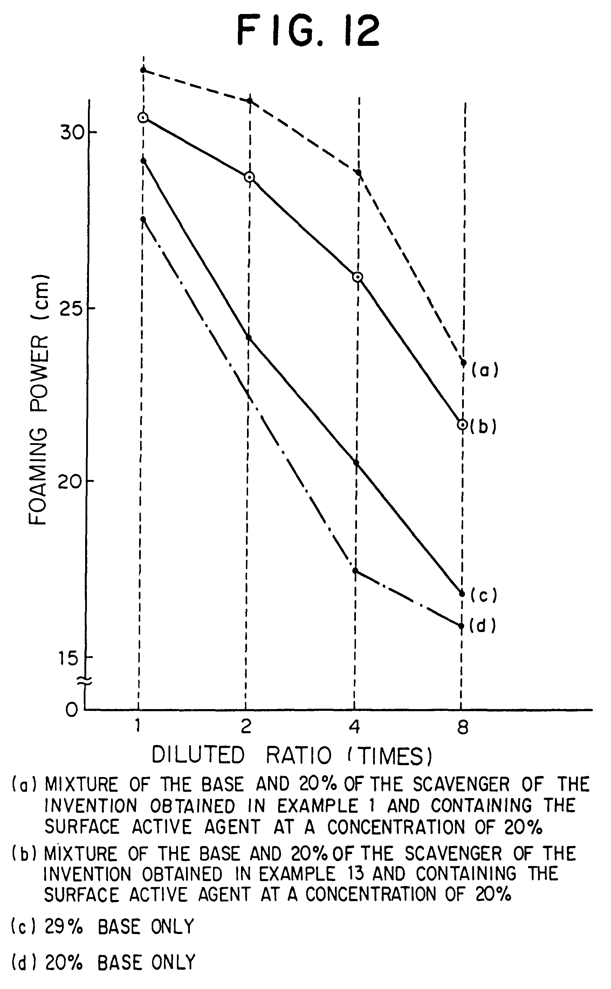

The scavenger of the present invention (water extract of rice) was mixed with a base prepared such that the concentration of a surface active agent became 29% or 20%, and then the concentration of the surface active agent was adjusted to 20%. As to the product obtained, a Leenerts test of detergent power, a plate-washing test, and a Ross-Miles test of foaming power were carried out and the results obtained are shown in Table 9 and Fig. 12.

The Ross-Miles test method and the Leenerts test method were carried out according to the JIS standard. The platewashing test method was carried out as follows. That is, a commercially available lard, a spond (110 mm x 75 mm x 30 mm), a plate (diameter 25 cm), and 20 g of each sample (10 g of each detergent + 10 g of city water) were prepared. First, 2,5 g of the commerically available lard was spread over the whole surface of one plate by a finger, and the plate was stored at about 25°C. The dry sponge was coated with 20 g of the sample, lightly crumpled to form foam, and the outside and the inside of the plate were each washed each once with the sponge. The washing operation was repeated 5 times for one plate, the test of how many plates could be washed until the sponge was not bubbled was carried out 5 times, and the mean value was determined. The results are shown in Table 9 below.

**Table 9**

| Concentration of Surface Agent | Sample Name | | Plate-washing Method (No. of plates) |
|---|---|---|---|
| 29% | Base only | 84.1 | 6.0 |
| 20% | Base only | 78.9 | 5.0 |
| 20% | Base + 20% product of invention in Example 1 | 89.0 | 7.2 |
| 20% | Base + 20% product of invention in Example 3 | 84.0 | 6.1 |
| Notes: The base of the mixture of polyoxyethylene lauryl ether sodium sulfate, coconut oil fatty acid amidopropylbetaine, laurylmethylamine oxide, and coconut oil fatty acid diethanol amide at a ratio of 21 : 7 : 2 : 3. The concentration of the surface active agent is the concentration of the final product. | | | |

As is clear by comparing the cases of using the base only as the surface active agent with each other, when the concentration of the surface active agent (base) is lowered from 29% to 20%, the detergency and the foaming power are greatly reduced. However, by adding the scavenger (product) of the present invention, even by lowering the concentration of the surface active agent, the detergency and the foaming power are superior to the case of the concentration of the surface active agent (base) only of 29%.

The scavenger of the present invention can be also used as a bath product. A bath product is used by adding it to bath water when taking a bath. When 50 ml of the scavenger of the present invention is added to 200 liters of warm water at 42°C and 100 subjects bathed, just after the bath, their skin were moist and also were smooth as compared with the case of bathing without using the scavenger of this invention.

Thus, for experimentally illustrating the warm bath effect (the effect of warming the human body completely and of being reluctant to chill after a bath) of the scavenger of the present invention, the skin surface temperature-maintaining effect after bathing was investigated using a thermography apparatus. In this test, 15 subjects were used and the mean values of the skin surface temperatures of the main test (using the scavenger of this invention of Comparative Example 1) read from the thermograph and the contrast test (using water only) are shown in Table 10 below.

**Table 10**

| (Mean value of skin surface temperature) | | | | | | | | | |
|---|---|---|---|---|---|---|---|---|---|
| | Before Immersion | After Immersion (Minute) | | | | | | | |
| | | 2 | 5 | 10 | 15 | 20 | 25 | 30 | 40 |
| (A) | 31.9°C | 35.0 | 34.6 | 34.5 | 34.2 | 33.9 | 33.6 | 33.3 | 32.7 |
| (B) | 31.9°C | 35.2 | 35.0 | 34.8 | 34.5 | 34.3 | 34.1 | 33.9 | 33.6 |
| (A): Bath of warm water only | | | | | | | | | |
| (B): Bath containing the scavenger of the invention | | | | | | | | | |

The measurement for the thermogram is now described.

In an inspection room at room temperature (20°C ± 1°C) and having no air flowing, the forearm of each subject was allowed to adapt to the room temperature in the exposed state of the arm for about 30 minutes. Thereafter, the infrared rays radiated from the human body were detected and the thermogram of the upper back portion of the right forearm of the subject was photographed using a thermographic apparatus for detecting the temperature information from the intensity of the infrared rays. Then, the right forearm of the subject was immersed up to the elbow in a water bath (9 liters) kept at 41°C for 10 minutes. The photographing intervals were 2, 5, 10, 15, 20, 25 and 40 minutes after finishing the immersion of the arm, whereby the change of the temperature of the skin surface with the passage of time was observed.

As is clear from the results shown in Table 10, in the measurement results using the forearm portion as the measuring division, the temperature difference between the case of taking the bath containing the scavenger of the present invention and the case of taking a bath of warm water only began to occur after 5 minutes and the temperature difference became 0.6°C after 30 minutes. Also, in the data of each subject with the passage of time, some immediately showed a temperature difference after 2 minutes and others showed a temperature difference after 10 to 15 minutes. However, all of them showed a temperature difference of from 0.5°C to 1.5°C after 40 minutes, which demonstrates a very remarkable warm-keeping effect of the scavenger of this invention.

Also, when the forearm of each subject was immersed in warm water containing a bath product mainly composed of sodium bicarbonate and Glauber's salt (which are not regarded as medicaments) and the same test as above was carried out, no temperature difference between the case of using a bath of warm water only was observed by the immersion of the forearm only.

The scavenger of the present invention can also be utilized as a preservative and freshness-keeping agent for foods.

An antimicrobial test of the scavenger of the present invention against Bacillus subtilis and Bacillus cereus (which are typical gram-positive bacteria causing the spoiling of e.g. boiled rice or bread), and Escherichia coli (which is a typical gram-negative bacterium and is considered to be a general indication of pollution) gave results as follows.

As a culture medium there was used 10 ml of an ordinary agar culture medium to which was added 1 ml of the scavenger of the present invention (the product obtained in Example 5).

As a control there was 10 ml of an agar cuture medium with 1 ml of water in place of the scavenger of this invention. The cultivation was carried out at 35°C and after 10 hours, 24 hours, 48 hours and 72 hours the state of growth of each bacterium was observed.

The results obtained are shown in Tables 11 to 13.

**Table 11**

| Bacillus subtilis | | |
|---|---|---|
| Cultivation Time | Scavenger of the Invention | Water |
| 10 hours | A | C |
| 24 hours | A | C |
| 48 hours | A | D |
| 72 hours | A | D |

**Table 12**

| Bacillus cereus | | |
|---|---|---|
| Cultivation Time | Scavenger of the Invention | Water |
| 10 hours | A | C |
| 24 hours | A | C |
| 48 hours | A | D |
| 72 hours | A | D |

**Table 13**

| Escherichia coli | | |
|---|---|---|
| Cultivation Time | Scavenger of the Invention | Water |
| 10 hours | A | C |
| 24 hours | A | D |
| 48 hours | A | D |
| 72 hours | B | D |

The meaning of the symbols in these tables is:
A: No growth
B: A little growth
C: Considerable growth
D: Great amount of growth

As is clear from the results in Tables 11 to 13, in the control culture medium, each bacterium grew greatly after the cultivation for 10 hours; while in the culture medium containing with the scavenger of the present invention, the growth of Bacillus subtilis and B. cereus was completely hindered even after 72 hours, and although growth of Escherichia coli was observed after cultivation of 48 hours or longer, in these cases, the scavenger of the invention showed a large antimicrobial effect as compared with the control.

The preservative effect of the scavenger of this invention on food was assessed using a steamed fish paste ("kamaboko") of the ground fish meat of a white fish having a large protein content, and a sweet beverage made from fermented rice ("amazake") which are respectively a popular Japanese food and drink.

First, to a 200 g portion of the ground fish meat was added the scavenger of the present invention obtained in Example 5 below in an amount of 1.0%, 0.1% or 0.01%. As controls, portions were used of the same ground fish meat with 1% water or 1% ethyl acetate added (although ethyl acetate was removed from the product in Example 5, under the supposition that some ethyl acetate might remain in the product, 1% thereof was used as a control.)

Each of the fish meat portions described was mashed well in an earthenware mortar which had been disinfected well with hot water and shaped into a suitable form. After steaming the meat in a steamer for 50 minutes, the shaped steamed meat was allowed to stand, on a piece of plastics wrap and lightly covered therewith, at room temperature, and the preservative effect of the additives was observed with the passage of time (days) on the staling (sometimes referred to as "threading"), the color, the freshness and the resilience thereof.

The staling results are shown in Table 14 below.

**Table 14**

| Additive | Amount (%) | Scavenger Concn. (ppm) | Elapsed Days | | | | | | | | | | | |
|---|---|---|---|---|---|---|---|---|---|---|---|---|---|---|
| | | | 1 | 2 | 3 | 4 | 5 | 6 | 7 | 8 | 9 | 10 | 11 | 12 |
| Water | 1.0 | - | a | a | b | d | e | e | e | e | e | e | e | e |
| Ethyl Acetate | 1.0 | - | a | a | c | d | e | e | e | e | e | e | e | e |
| Product of the invention | 1.0 | 450 | a | a | a | a | a | a | a | a | b | b | b | b |
| | 0.1 | 45 | a | a | a | a | a | a | b | b | b | b | b | b |
| | 0.01 | 4.5 | a | a | a | a | a | b | d | d | e | e | e | e |

The symbols in the table refer to the observed staling ("threading"):
- a:: none
- b:: very slight
- c:: slight
- d:: extensive
- e:: great

As shown in Table 14, in the controls with water or ethyl acetate, slight staling was already observed after 3 days and it was complete after 4 days, but in the product with the scavenger of the invention, the forming of very slight staling was observed only after 6 days in the case of adding 0.01% the scavenger of this invention, which showed the addition of 0.01% of the scavenger had the effect of prolonging the storage life by 2 days (complete threading was observed after 7 days). Also, it can be seen that in the product with 1.0% or 0.1% the scavenger of the present invention, very slight staling was observed after 9 days in the case of adding 1.0% and after 7 days in the case of adding 0.1%, which showed that the preservative effect of the scavenger was very high. In addition, in the latter products, complete staling was not observed during the 12 days of the experiment.

The bacteria causing staling were identified as of the genus Bacillus; so the scavenger of the invention was effective against putrefying bacteria.

In products such as ground fish meat, it is useful that the occurence of putrefaction is delayed even for one day, and since by using the scavenger of the present invention the occurrence of putrefaction is prolonged to 4 days even by the addition of 0.1% thereof, this confirms the high usefulness and effectiveness of the scavenger.

Also, in the control samples and those with only 0.01% of the scavenger of the invention, growth of mold was observed on the surfaces thereof after 8 days, while when 0.1 or 1.0% of the scavenger was added, growth of mold was not observed after 8 days. However, even in the samples with 0.1% of the scavenger of the invention, growth of mold was first observed after 9 days and with 1.0% of the scavenger only first after 11 days.

Furthermore, when these samples were allowed to stand as they were, in the control sample with 1% water added, a large amount of colonies of green-spored Penicillium having a diameter of from 4 to 8 mm and (1.0 x 2.3 cm) of Mucor grew, in the control sample with 1% ethyl acetate, colonies (1.5 x 2.0 cm) of white Mucor, colonies (1.5 x 1.8 cm) of Penicillium of deep-green spores, colonies of Koji molds (Aspergillus) of yellow spores, and a large number of colonies of blue Penicillium of green spores grew; in the sample with 0.01% added scavenger of Comparative Example 4 of this invention, colonies (1.5 x 4.3 cm) of Mucor, two colonies (1.0 x 0.8 cm) of Aspergillus, and a large number of colonies of Penicillium grew, and in the sample with 0.1% the said scavenger of this invention, colonies (1.2 x 3.0 cm) of Mucor, colonies (diameter: 0.8 cm) of Aspergillus, and a large number of colonies of Penicillium of green spores grew, while in the sample containing the 1.0% of the scavenger of this invention, only two colonies (diameter: about 4 mm) of Penicillium grew.

The above results show that the scavenger of the present invention has an antimicrobial activity against bacteria and also molds.

Also, the effect of the scavenger of the invention was determined on the quality keeping of "kamaboko" fish meat which was again placed on plastic film and lightly covered with film. The results are shown in Table 15 below.

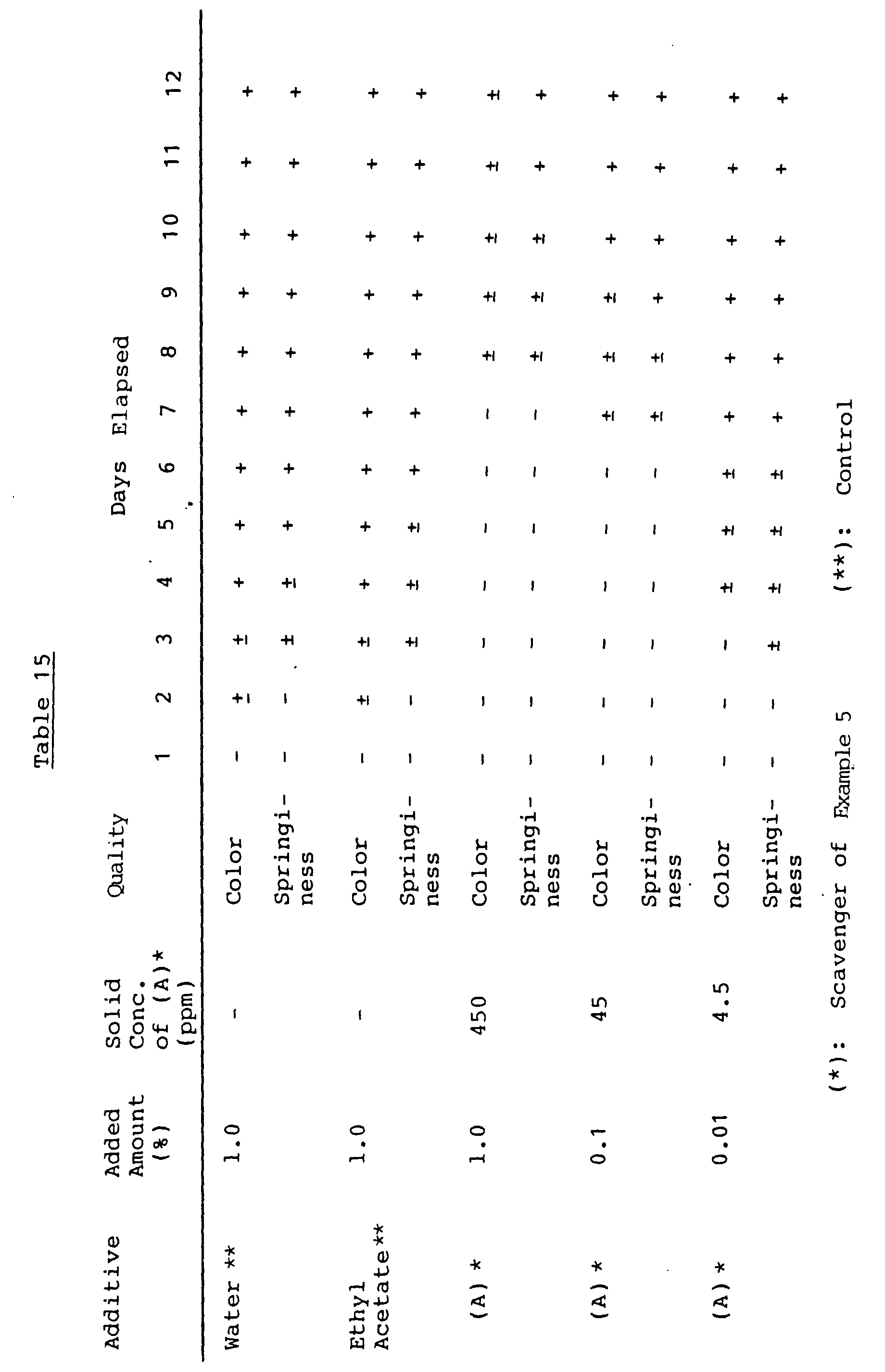

In Table 15 the symbols have the following meanings:
color:
   - white
   ± slightly brown
   + brown
Springiness:
   - High springiness
   ± Slightly inferior springiness
   + No springiness

As shown in Table 15, in the controls not containing the scavenger of the present invention, when the food was stored at room temperature without completely sealing it, its springiness (i.e. flexibility) already becomes inferior after 2 days and its commercial value is lost, while in the samples containing the scavenger of Comparative Example 4 of this invention, the sample with 0.1% of scavenger keeps its springiness and freshness until the 6th day and the sample with 1.0% of scavenger keeps it springiness and freshness until the 7th day, which shows that the scavenger of the invention has a remarkable food quality keeping effect. In addition, with the increase of the springiness of the food, the food exhibits more freshness, which shows that there is a correlation between the springiness and the freshness of food.

Also, as to the color of the surface of the food, as shown in Table 15, it can be seen that in the sample with 0.01% of the scavenger of this invention, the occurence of browning is delayed 2 days and in the samples with 0.1% and 1.0% of the scavenger, browning is delayed 5 to 6 days.

From these results described above, it has been found that for the current requirements for storage of food, in particular, for keeping an additive-free food in a fresh state, the preservative effect of the scavenger of this invention is very effective. In particular, for a food wherein the freshness is most important, and having a short usable life, such as "kamaboko" used in the foregoing experiments, the prolonging by one day of the keeping time is important in the production, transport, sale and consumption of the goods, and it can be said to be socially very useful that the food-keeping effect is prolonged several days by using the scavenger of the present invention.

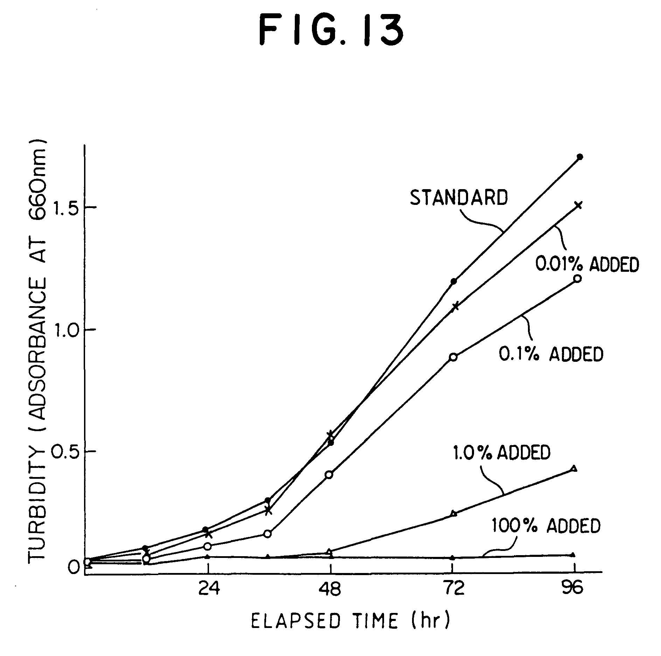

Then, on an unsterilized sweet beverage made from fermented rice and called in Japan "amazake", which has a large glucide content, the keeping effect of the scavenger of the present invention was determined on added amounts of 10.0%, 1.0%, 0.1% or 0.01% of the scavenger of this invention as obtained in Comparative Example 1. The results thereof with the passage of time are shown in Fig. 13. For measuring the turbidity of the beverage, solid matter was removed therefrom by filtration and the measurement was carried out in an open system in a wide-mouthed bottle.

In a control beverage sample without added scavenger of this invention, tubidity began to increase after 12 hours and after allowing it to stand for 24 hours, the absorbance at 660 nm became 0.170, which showed a considerable increase of turbidity. In the sample with only 0.01% of the scavenger of this invention, a significant difference was not observed as compared with the control sample; but in the sample with 0.1% the scavenger of this invention, increase of turbidity was not observed after allowing to stand it for 12 hours and also growth of bacteria since then was clearly restrained as compared with the control sample. Microscopic examination showed the turbidity to be due to a yeast. In the sample with 1.0% of the scavenger, too much increase of turbidity was not observed after allowing it to stand it for 48 hours and the growth of bacteria since then was considerably restrained. Thus by the addition of 1.0% of the scavenger of this invention to the beverage, a preservative effect for at least 36 hours was obtained and thus the scavenger of this invention was very effective.

Also, when the pH of these beverage samples was measured, in the control sample the pH became lower than 3.0 after allowing to stand for 72 hours, while in the sample with 1.0% of the scavenger the pH was not changed much after allowing to stand for 72 hours and the pH became 5.7 even after allowing to stand for 96 hours. By the change of pH, in the control sample not containing the scavenger, the increase of turbidity by staining with microorganisms was confirmed and also in the sample with 1.0% the scavenger of this invention prolonging of the quality-keeping time by longer than 2 days was confirmed.

Also, in the beverage not containing added scavenger or with 0.1% or 0.01% of the scavenger, the formation of a film-like matter on the surface of the liquid was observed after 2 days. However, in the beverage with 1.0% or more of the scavenger of this invention, the formation of such a surface film was not observed up to 4 days. By observation with a microscope, the surface film was found to be a yeast, and so the results described above confirm that the scavenger of the invention has an antimicrobial activity to yeast. Because such inhibition effect was confirmed in the sweet beverage which is very liable to be putrefied and could be a culture medium for microorganisms, it was confirmed that the scavenger of the present invention was very excellent for practical use.

Also, in the sample with 10% of added scavenger of the present invention, the sample was not stained by microorganisms and no turbidity occurred during testing for 4 days. In addition, when the change of the sample was further observed after the test, no turbidity was observed even after 12 days. Thus, it was confirmed that the food-keeping effect of the scavenger of this invention for the "amazake" beverage is very remarkable. As to the odor of the sample, the sample gave no acid smell.

Furthermore, when the pH of the sample with 10% of the scavenger was measured, no change of pH was observed after 5 days and thus it was also confirmed from the pH that the sample was not putrefied.

This shows surprisingly, that in the beverage containing 10% of the scavenger of this invention, the growth of microorganisms could be prevented for longer than 12 days.

Accordingly, it is clear that the scavenger of the present invention has a food-keeping (putrefaction-inhibition) effect. As described above, in a sweet beverage which is not heat-sterilized arid is liable to putrefy, the life as goods can be prolonged longer than 1.5 days (36 hours) and this is a large antiseptic and antimicrobial effect and shows the effectiveness of the scavenger of this invention as a preservative.

Furthermore, the scavenger of the present invention is effective as a freshness-keeping agent for vegetables, fishes, etc. The result of carrying out a test by spraying the scavenger of this invention onto a lettuce is as follows.

When water was sprayed onto a lettuce and the lettuce was allowed to stand at room temperature, the lettuce was bent after 12 hours and the wound became browned after 2 days. However, in a lettuce sprayed with the scavenger of Comparative Example 1 diluted 50 times with water, the lettuce kept its freshness even after 20 hours and was not discolored up to the 4th day.

Now, the invention is illustrated by the following examples, wherein the parts and percentages are by weight.

### Comparative Example 1

After grinding well 15 kg of polished rice, 45 liters of warm water at 60°C and 50 g of liquefying amylase were added thereto followed by stirring well. Thereafter, the temperature of the mixture was gradually raised to boiling, and after extracting for 5 minutes, the extract was cooled to 30°C. Thereafter, the mixture was pressed in a pressing apparatus, and 41 liters of a pressed out liquid (the scavenger of this invention) and 16 kg of a residue were obtained.

### Comparative Example 2

After grinding well 1 kg of polished rice, 5 liters of an aqueous 0.1% hydrochloric acid solution was added thereto followed by stirring well and the mixture was allowed to stand for 6 hours. Thereafter, the mixture was pressed in a pressing apparatus to provide 4.6 liters of a pressed out liquid and 1.2 kg of a residue. By neutralizing this liquid with an aqueous 1N sodium hydroxide solution, 4.7 liters of the scavenger of this invention were obtained.

### Comparative Example 3

After grinding 1 kg of polished rice, 3 liters of 95% ethanol was added thereto followed by stirring well and the mixture was allowed to stand for 4 days. Thereafter, the mixture was pressed in a pressing apparatus to provide 2.5 liters of a pressed out liquid and 1.2 kg of a residue. To one liter of this liquid was added 500 ml of water and then ethanol was completely removed therefrom by means of a rotary evaporator to provide 480 ml of the scavenger of this invention.

### Example 1

After adding 10 ml of a cultured yeast solution to one liter of the water extract obtained in Comparative Example 1 and the mixture was kept at a temperature of 25°C to carry out the alcoholic fermentation for 4 days. Thereafter, the fermented product was filtered to provide 940 ml of the scavenger (product) of this invention.

### Comparative Example 4

The pressed liquid obtained in Comparative Example 1 was acidified by the addition of hydrochloric acid and the acidified product was extracted with ethyl acetate to remove dextrin. Thereafter, the ethyl acetate was volatilized off and the residue was dissolved in water to provide 750 ml of the scavenger of this invention.

### Comparative Example 5

After mixing 10 liters of the product of this invention obtained in Comparative Example 4 and 6 kg of dextrin, the mixture was spray-dried to provide 7 kg of a powder. The powder was useful as an anti-ulcer agent and a preservative.

### Comparative Example 6

A mixture of 30 parts of the product of this invention obtained in Comparative Example 4, 2.0 parts stearic acid, 0.5 parts cetanol, 2.0 parts lanolin, 2.0 parts isopropyl myristate, 3.0 parts squalene, 8.0 parts liquid paraffin, 1.7 parts polyoxyethylene cetyl ether, 0.8 parts sorbitan monostearate and 0.2 parts vitamin A acetic acid ester was heated to about 75°C to form a solution and then 1.0 parts triethanolamine, 4.0 parts glycerol, 0.3 part a perfume and antiseptic, and 44.5 parts water was added to the solution by heating to about 75°C with stirring to provide a milk lotion for use on the skin.

### Comparative Example 7

To a mixture of 30.0 parts of the product obtained in Comparative Example 1,3.0 parts of sorbitol, 5.0 parts glycerol, and 41.0 parts water were added 0.1 part allantoin, 0.5 part a polyoxyethylene-cured castor oil derivative, 20.0 parts ethanol and 0.4 parts of a perfume with stirring to form a homogeneous solution. The solution obtained can be used as a facial lotion.

### Comparative Example 8

A mixture of 15.0 parts beeswax, 31.0 parts petroleum jelly, 20.0 parts fluid paraffin, 4.0 parts sorbitan sesquioleate and 2.0 parts ethyl p-aminobenzoate was heated to about 75°C to form a solution and then 10.0 parts of the product obtained in Comparative Example 1, 1.0 parts of a perfume, 0.4 parts of an antioxidant and antiseptic, and 16.6 parts of water were added to the solution by heating to about 75°C with stirring followed by cooling to provide a cosmetic sunscreen.

### Comparative Example 9

After dissolving 29.9 parts polyethylene glycol 4000 in 29.9 parts polyethylene glycol 400 by heating to 65°C on a hot-water bath, 40.0 parts of the product of this invention obtained in Comparative Example 1 and 0.2 parts of an acrynol fine powder were added to the solution, followed by stirring and cooling to provide an ointment.

### Comparative Example 10

A mixture of 24.0 parts of myristic acid, 2.0 parts stearic acid K, 10.0 parts N-lauroylmethyltaurine sodium, 2.0 parts polyoxyethylene sorbitan tetraoleate, 4.0 parts coconut oil fatty acid, 2.0 parts avocado oil and 10.0 parts glycerol was heated to 75°C to form a solution which was put into a mixing tank. The pressure in the tank was reduced to not higher than 93 x 10³ Pa (70 cm Hg) and an aqueous solution prepared by dissolving 5.5 parts potassium hydroxide in 19.0 parts of purified water in a dissolver by heating to 50°C was added to the solution to carry out a saponification.

Thereafter, 11.2 parts of purified water and 10.0 parts of the product of this invention obtained in Comparative Example 1 were added to the saponified mixture followed by cooling and stirring, 0.3 parts of a perfume was added thereto at 50°C, and after cooling the mixture to 35°C, stirring was stopped. The mixture was kept at 40°C for 48 hours to carry out curing, to provide a face-washing foam.

### Comparative Example 11

A mixture of 21 parts polyoxyethylene lauryl sodium sulfate, 7 parts coconut oil fatty acid amide propylbetaine, 2 parts lauryl dimethylamine and 3 parts coconut oil fatty acid diethanolamide was heated to 70°C with stirring to form a transparent liquid. Then. 20 parts of the product of this invention obtained in Comparative Example 1 and 47 parts purified water were added to the liquid and the mixture was cooled to 30°C with stirring to provide a kitchen detergent.

### Comparative Example 12

A mixture of 10 parts of sodium hydrogenated glyceryl cocoate sulfate, 10 parts of polyoxyethylene tris alkyl ether sodium acetate, 20 parts of 2-alkyl-N-carboxymethyl-N-hydroxyethylimidazolium betaine, 0.2 parts of an alkyl para-hydroxybenzoate and 30 parts of purified water was heated to 70°C with stirring to form a transparent liquid. Then, after adding thereto 15 parts of the product of this invention obtained in Comparative Example 1, the resultant mixture was allowed to cool, 0.5 parts of a perfume and 14.3 parts of purified water were added thereto at 45°C, and the resultant mixture was stirred for about one hour at room temperature, to provide a soap for use on the body.

### Comparative Example 13

After melting 1 part of polyoxyethylene hydrogenated castor oil by heating to 60°C, 1 part of a perfume was added thereto followed by _stirring well, and 98 parts of the product of this invention obtained in Comparative Example 1 was added to the mixture followed by stirring, to provide a bath product.

## Claims

1. A method of preparing a scavenger of active oxygen, which comprises the steps of:
grinding polished rice,
adding warm water and liquefying amylase to the ground polished rice,
stirring the mixture well,
gradually raising the temperature of the mixture to boiling,
extracting under boiling,
cooling the extract,
pressing the mixture in a pressing apparatus,
adding a cultured yeast solution to the pressed out extract,
keeping the mixture at a constant temperature to carry out alcoholic fermentation, and
filtering the fermented product.

2. A medicament, cosmetic or food containing a scavenger of active oxygen prepared according to the method in Claim 1.

## Patentansprüche

1. Verfahren zur Herstellung eines Sauerstoffaufnehmers, umfassend die folgenden Schritte:
Mahlen von poliertem Reis,
Zufügen von warmem Wasser und sich verflüssigender Amylase zu dem gemahlenen polierten Reis,
sorgfältiges Umrühren der Mischung,
allmähliche Erhöhung der Temperatur der Mischung bis zum Kochen,
Extrahieren während des Kochens,
Abkühlen des Extrakts,
Auspressen der Mischung in einer Pressapparatur,
Zufügen einer gezüchteten Hefelösung zu dem ausgepressten Extrakt,
Aufbewahren der Mischung bei konstanter Temperatur, um eine alkoholische Gärung auszuführen, und
Filtern des gegorenen Produkts.

2. Medikament, Schönheits- oder Nahrungsmittel, enthaltend einen Sauerstoffaufnehmer hergestellt gemäß dem Verfahren von Anspruch 1.

## Revendications

1. Procédé de préparation d'un agent d'élimination de l'oxygène actif, comprenant les étapes consistant à :
moudre du riz poli,
ajouter de l'eau chaude et de l'amylase liquéfiante au riz poli moulu,
bien brasser le mélange,
augmenter progressivement la température du mélange jusqu'à ébullition,
extraire sous ébullition,
refroidir l'extrait,
presser le mélange dans un appareil de pressage,
ajouter une solution de levure cultivée à l'extrait pressé,
maintenir le mélange à une température constante pour réaliser une fermentation alcoolique, et
filtrer le produit fermenté.

2. Médicament, cosmétique ou aliment contenant un agent d'élimination de l'oxygène actif préparé par le procédé selon la revendication 1.
